# EUROPEAN PATENT APPLICATION

(11) **EP 4 834 194 A1**
(43) Date of publication of application: **04.11.2026**
(21) Application number: 24910507.3
(22) Date of filing: 28.11.2024
(51) Int. Cl.: A61B 1/00, A61B 1/06

(54) **ENDOSCOPE AND DISTAL MODULE THEREOF**

(30) Priority: 29.12.2023 CN 202311871630
(71) Applicant: Guangzhou Red Pine Medical Instrument Co., Ltd., Guangzhou, Guangdong 510000 (CN)
(72) Inventor: YI, Feng, Guangzhou, Guangdong 510000 (CN); LI, Jing, Guangzhou, Guangdong 510000 (CN); MA, Qiang, Guangzhou, Guangdong 510000 (CN); HUANG, Jiazi, Guangzhou, Guangdong 510000 (CN); ZHOU, Qili, Guangzhou, Guangdong 510000 (CN)
(74) Representative: Zacco Sweden AB
(86) International application number: PCT/CN2024/135181
(87) International publication number: WO 2025/139574

(57) **Abstract**

An endoscope and a distal module (10) thereof. The distal module (10) comprises an end cap (11) and a light-emitting element (14). The end cap (11) is provided with a light-concentrating structure (112) running through the end cap. A first opening (1121) and a second opening (1122) are respectively formed at two ends of the light-concentrating structure (112), the radial size of the light-concentrating structure (112) gradually decreases in the direction from the first opening (1121) to the second opening (1122), and a reflection film is provided on a surface of the light-concentrating structure (112). The light-emitting element (14) is arranged in the light-concentrating structure (112) and is provided with a first surface (141) facing the first opening (1121) and a second surface (142) facing the second opening (1122), wherein the second surface (142) is higher than the second opening (1121) in the direction from the second opening (1122) to the first opening (1121). By means of such an arrangement, the illumination beam angle of the distal module (10) can be reduced, such that the illumination beam angle is closer to the shooting field of view of a camera, thereby sufficiently using light emitted from the light-emitting element to supplement light to the camera, and improving the utilization efficiency of light energy.

## Description

### TECHNICAL FIELD

The present application relates to the field of medical instrument technologies, and in particular, to an endoscope and a distal end module thereof.

### BACKGROUND

An endoscope is configured to visually inspect difficult-to-reach positions such as organs in a patient to observe lesion conditions. Conventionally, the endoscope includes an insertion portion, a handle is arranged at an end of the insertion portion adjacent to an operator, and a distal end module is arranged at an end of the insertion tube away from the operator. The distal end assembly generally includes a camera and light-emitting elements arranged on two opposite sides of the camera. The light-emitting elements can illuminate a lesion region to facilitate the camera to capture an image of the lesion region. However, a beam angle resulting from the superposition of the two light-emitting elements is generally much greater than a shooting field of view of the camera, leading to light energy loss and a low light energy utilization efficiency.

### SUMMARY

Based on this, in order to address the problem of the low light energy utilization efficiency of the distal end module of the endoscope, an endoscope and a distal end module thereof are provided.

A distal end module is used in an endoscope, and the distal end module includes:
an end cover provided with a light-focusing structure extending through the end cover, a first opening and a second opening being formed at two ends of the light-focusing structure respectively, a radial size of the light-focusing structure being gradually reduced in a direction from the first opening to the second opening, and a reflecting film being arranged on a surface of the light-focusing structure; and
a light-emitting element arranged in the light-focusing structure and having a first surface facing the first opening and a second surface facing the second opening, the second surface being higher than the second opening in a direction from the second opening to the first opening.

In the above distal end module, the light-focusing structure and the reflecting film are arranged on the end cover and cooperate to focus light emitted by the light-emitting element, which can reduce a lighting beam angle of the distal end module to cause the lighting beam angle to more closely conform to the shooting field of view of the camera, thereby fully utilizing the light emitted by the light-emitting element to supplement the camera with light, improving the light energy utilization efficiency. Meanwhile, the light is converged by means of the light-focusing structure on the end cover, the light-focusing structure is highly integrated with the distal end module, an arrangement process is simple, an arrangement cost is low, and a space occupied by the light-focusing structure is small. Compared with structures such as a reflector, the light-focusing structure can be better adapted to the distal end module with a small size, reduce an injury to a patient caused by the endoscope, while accommodating the focusing of the light-emitting element having lower power in the distal end module. Furthermore, by gradually reducing the radial size of the light-focusing structure, distribution of a light field can be effectively regulated and controlled, which is conducive to converging the light towards a center of the light field to improve central illuminance and a light output efficiency of the lighting light field, and meanwhile, an edge field of view can maintain sufficient illuminance and good uniformity, thus effectively improving a lighting effect.

In one of the embodiments, a distance between the first opening and the second opening is greater than or equal to a thickness of the end cover.

In one of embodiments, the end cover has a top cover portion, a side cover portion and a raised portion. The side cover portion is connected to a side of the top cover portion and connected to a part of a contour of the top cover portion. The raised portion is connected to a side of the top cover portion facing the side cover portion. The light-focusing structure is positioned corresponding to the raised portion and extends through the top cover portion and the raised portion.

In one of the embodiments, a shape of a section of the light-focusing structure on a plane perpendicular to an axial direction of the distal end module is an irregular shape, an axisymmetric shape, a centrosymmetric shape or a rotationally symmetric shape.

In one of the embodiments, a shape of a section of the light-focusing structure on a plane parallel to an axial direction of the distal end module is a flat surface or a curved surface.

In one of the embodiments, a shape of a section of the light-focusing structure on a plane parallel to an axial direction of the distal end module is flat, a shape of a section of the light-focusing structure on a plane perpendicular to the axial direction is rectangular, and an included angle between a contour of the section of the light-focusing structure on the plane parallel to the axial direction and the axial direction is greater than or equal to 35° and less than or equal to 51°. Alternatively,
the shape of the section of the light-focusing structure on the plane parallel to the axial direction is flat, the shape of the section of the light-focusing structure on the plane perpendicular to the axial direction is circular, and the included angle between the contour of the section of the light-focusing structure on the plane parallel to the axial direction and the axial direction is greater than or equal to 36.3°.

In one of the embodiments, a shape of a section of the light-focusing structure on a plane parallel to an axial direction of the distal end module is a concave arc surface. When a ratio between a radial size of the first opening and a radial size of the second opening ranges from 1.55 to 2, a curvature of a contour of the section of the light-focusing structure on the plane parallel to the axial direction is greater than or equal to 1.5 and less than or equal to 10. When the ratio between the radial size of the first opening and the radial size of the second opening ranges from 2.1 to 2.34, the curvature of the contour of the section of the light-focusing structure on the plane parallel to the axial direction is greater than or equal to 1 and less than or equal to 2.8. When the ratio of the radial size between the first opening and the radial size of the second opening ranges from 2.35 to 2.58, the curvature of the contour of the section of the light-focusing structure on the plane parallel to the axial direction is greater than or equal to 2 and less than or equal to 5.

In one of the embodiments, a distance between the first opening and the second opening is greater than or equal to 0.5 mm and less than or equal to 2 mm.

In one of the embodiments, a ratio of edge illuminance to central illuminance, at a working distance, of a lighting light field of the light-emitting element passing through the light-focusing structure is greater than or equal to 0.5% and less than or equal to 5%.

In one of the embodiments, a size of a section of the light-emitting element on a plane perpendicular to an axial direction of the distal end module is less than a size of a section of the second opening of the light-focusing structure on the plane perpendicular to the axial direction.

In one of the embodiments, the reflecting film covers a surface of the light-focusing structure and a surface of the end cover corresponding to the first opening; and/or
a material of the reflecting film includes aluminum, silver or chromium.

In one of the embodiments, the distal end module further includes a camera. The end cover is further provided with a light-receiving hole. The camera is arranged corresponding to the light-receiving hole. The end cover is provided with two light-focusing structures. The two light-focusing structures are arranged on two opposite sides of the light-receiving hole respectively. The light-emitting element is arranged in each of the two light-focusing structures.

In one of the embodiments, the distal end module further includes a light-transmitting sealing structure. The light-transmitting sealing structure is arranged in the light-focusing structure and seals a side of the light-emitting element facing the first opening.

An endoscope includes an insertion portion and the distal end module according to any one of the above embodiments. The distal end module is arranged at an end portion of the insertion portion.

### BRIEF DESCRIPTION OF THE DRAWINGS

FIG. 1 is a schematic structural view of a distal end module in some embodiments.
FIG. 2 is a schematic structural view of an end surface of the distal end module shown in FIG. 1.
FIG. 3 is a schematic sectional view of the distal end module shown in FIG. 2 taken along direction A-A.
FIG. 4 is a schematic partial enlarged view of region D of the distal end module shown in FIG. 3.
FIG. 5 is a schematic structural view of an end surface of a distal end module in some other embodiments.
FIG. 6 is a schematic sectional view of the distal end module shown in FIG. 5 taken along direction B-B.
FIG. 7 is a schematic structural view of an end surface of a distal end module in still other embodiments.
FIG. 8 is a schematic partial enlarged view of a section of the distal end module shown in FIG. 7 taken along direction C-C.
FIG. 9 shows schematic diagrams of lighting effect of a distal end module in a first embodiment of the present application.
FIG. 10 shows diagrams of lighting effect of a conventional distal end module without a light-focusing structure.
FIG. 11 shows schematic diagrams of lighting effect of a distal end module according to a second embodiment of the present application.
FIG. 12 shows schematic diagrams of lighting effect of a distal end module according to a third embodiment of the present application.
FIG. 13 shows schematic diagrams of lighting effect of a distal end module according to a fourth embodiment of the present application.
FIG. 14 shows diagrams of lighting effect of another conventional distal end module without the light-focusing structure.
FIG. 15 shows illuminance distribution diagrams of distal end modules according to a fifth embodiment and a sixth embodiment of the present application.
FIG. 16 shows illuminance distribution diagrams of the distal end modules according to the sixth embodiment and a seventh embodiment of the present application.
FIG. 17 shows illuminance distribution diagrams of distal end modules according to an eighth embodiment and a ninth embodiment of the present application.
FIG. 18 sequentially shows diagrams of lighting effects of distal end modules according to a tenth embodiment, an eleventh embodiment and a twelfth embodiment of the present application.
FIG. 19 sequentially shows diagrams of lighting effects of distal end modules according to a thirteenth embodiment, a fourteenth embodiment and a fifteenth embodiment of the present application.
FIG. 20 sequentially shows diagrams of lighting effects of distal end modules according to a sixteenth embodiment, a seventeenth embodiment and an eighteenth embodiment of the present application.

Illustration for Reference signs:
10: distal end module; 11: end cover; 111: end surface; 112: light-focusing structure; 1121: first opening; 1122: second opening; 113: light-receiving hole; 114: top cover portion; 115: side cover portion; 116: raised portion; 12: sleeve; 121: instrument hole; 13: camera; 14: light-emitting element; 141: first surface; 142: second surface; 15: flexible circuit board; 16: bracket; 17: transmission line; 18: light-transmitting sealing structure; 19: photosensitive element.

### DETAILED DESCRIPTION

In order to make the aforementioned objects, features and advantages of the present application more apparent, the embodiments of the present application are described below in detail with reference to the accompanying drawings. In the following description, numerous specific details are set forth, so as to provide a thorough understanding of the present application. However, the present application may be implemented in many ways different from those described herein, those skilled in the art may make similar improvements without departing from the essence of the present application, and therefore, the present application is not limited to the specific examples disclosed below.

In descriptions of the present application, it should be understood that, if terms "center", "longitudinal", "transverse", "length", "width", "thickness", "upper", "lower", "front", "rear", "left", "right", "vertical", "horizontal", "top", "bottom", "inner", "outer", "clockwise", "anticlockwise", "axial", "radial", "circumferential" etc. appear, directions or positional relationships indicated by these terms are based on orientations or positional relationships shown in the accompanying drawings, and they are used only for describing the present application and for description simplicity, but do not indicate or imply that an indicated device or element must have a specific orientation or be constructed and operated in a specific orientation. Therefore, it cannot be understood as a limitation on the present application.

In addition, if the terms such as "first" and "second" appear, they are only for purposes of description and cannot be understood as indicating or implying relative importance or implicitly indicating a quantity of the indicated technical features. Thus, the feature defined with "first" and "second" may include at least one of the features explicitly or implicitly. In the description of the present disclosure, if the term "a plurality of" appears, it means at least two, such as two, three, etc., unless otherwise clearly and specifically limited.

In the present application, unless explicitly specified or limited otherwise, if terms "mounted", "connected", "coupled", and "fixed" and the like appear, these terms are understood broadly. It may indicate, for example, fixed connections, detachable connections, or integral connections; may indicate mechanical or electrical connections; may indicate direct connections or indirect connections via intervening mediums; may indicate communication or an interaction relationship between two elements, unless otherwise explicitly specified. For a person of ordinary skill in the art, the specific meanings of the above terms in the present application can be understood according to the specific circumstances.

In the present application, unless explicitly specified or limited otherwise, the description that a first feature is "on" or "below" a second feature may mean that the first feature is in direct contact with the second feature, or the first feature is in contact with the second feature via an intervening feature formed therebetween. Furthermore, a first feature "on," "above," or "on top of" a second feature may mean that the first feature is right or obliquely "above" the second feature, or just mean that the first feature is at a height in level higher than that of the second feature. while a first feature "below," "under," or "underneath" a second feature may mean that the first feature is right or obliquely "below" the second feature, or just mean that the first feature is at a height in level lower than that of the second feature.

It should be noted that when an element is referred to as being "fixed on" or "provided at" another element, the element may be directly located on the another element or an intermediate element may exist. If one element is considered to be "connected" to another element, it may be directly connected to the another element or an intermediate element may co-exist. If exists, the terms "vertical", "horizontal", "upper", "lower", "left", "right", or the like, used in this application are for purposes of illustration only and do not denote a sole embodiment.

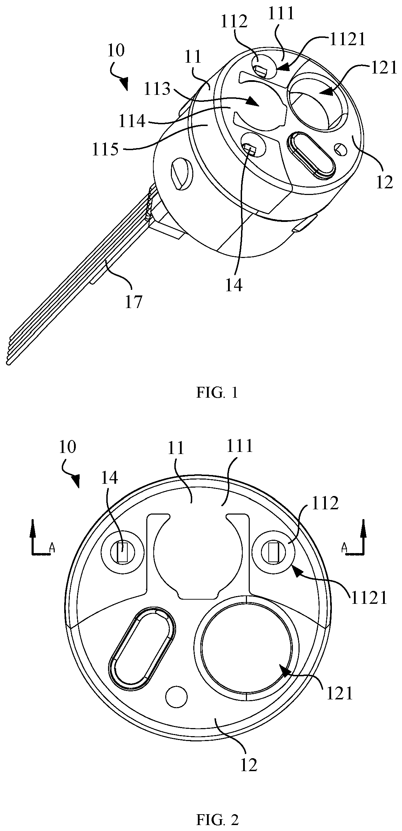

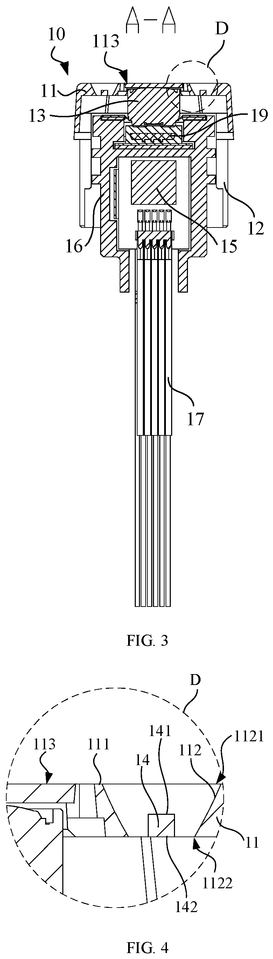

Referring to FIG. 1, FIG. 2 and FIG. 3, FIG. 1 shows a schematic structural view of a distal end module 10 in some embodiments, FIG. 2 shows a schematic structural view of an end surface 111 of the distal end module 10 in some embodiments, and FIG. 3 shows a schematic sectional view of the distal end module 10 shown in FIG. 2 taken along direction A-A. The distal end module 10 according to the present application may be applied in an endoscope. A camera 13 and a light-emitting element 14 are mounted on the distal end module 10. The endoscope may further include a handle and an insertion portion. Two ends of the insertion portion are connected to the handle and the distal end module 10 respectively. In use of the endoscope, the insertion portion can extend into the body of a patient, the light-emitting element 14 can emit light to illuminate a lesion region to supplement the camera 13 with light, and the camera 13 is configured to acquire an image of the lesion region to facilitate diagnosis or treatment. The light-emitting element 14 includes, but is not limited to, an LED. The camera 13 may include one or more lenses having optical power. The distal end module 10 may further include a photosensitive element 19 arranged on a light output side of the camera 13. The photosensitive element 19 includes, but is not limited to, a charge coupled device (CCD) or a complementary metal-oxide semiconductor (CMOS) sensor. The camera 13 can receive the light from the lesion region. The light is adjusted by the camera 13 and then emitted to the photosensitive element 19 for photoelectric conversion, so as to output image information of the lesion region.

As shown in FIG. 4, FIG. 4 is a schematic partial enlarged view of region D of the distal end module 10 shown in FIG. 3. In some embodiments, the distal end module 10 includes an end cover 11 and a sleeve 12 connected to each other. The camera 13 and the photosensitive element 19 are accommodated in a space defined by the end cover 11 and the sleeve 12. The end cover 11 is provided with a light-focusing structure 112 extending through the end cover 11. It can be understood that the light-focusing structure 112 is formed by a side wall of a groove extending through the end cover 11. A first opening 1121 and a second opening 1122 are formed at two ends of the light-focusing structure 112 respectively. The end cover 11 may have an end surface 111 configured for facing the lesion region. The first opening 1121 may be formed at the end surface 111 of the end cover 11 by the light-focusing structure 112. The distal end module 10 may have an axial direction parallel to a direction from the first opening 1121 to the second opening 1122. The axial direction is parallel to an optical axis of the camera 13. The first opening 1121 and the second opening 1122 may be understood to be formed by the two positions on the light-focusing structure 112 that are farthest apart in a plane perpendicular to the axial direction. A radial size of the light-focusing structure 112 gradually decreases in the direction from the first opening 1121 to the second opening 1122 along the axial direction of the distal end module 10. A surface of the light-focusing structure 112, i.e., the side wall of the groove structure extending through the end cover 11 for forming the light-focusing structure 112, is provided with a reflecting film (not shown). The light-emitting element 14 is arranged in the light-focusing structure 112, and has a first surface 141 facing the first opening 1121 and a second surface 142 facing the second opening 1122. The second surface 142 is higher than the second opening 1122 in a direction from the second opening 1122 to the first opening 1121. In other words, the light-emitting element 14 is arranged between the first opening 1121 and the second opening 1122, and there is a height difference between the second surface 142 of the light-emitting element 14 and the second opening 1122 in the axial direction.

It should be noted that the first surface 141 of the light-emitting element 14 may be a light-emitting surface of the light-emitting element 14, and then, the first opening 1121 also serves as a light outlet of the light-emitting element 14. Light emitted from the first surface 141 has a divergence angle. When the light emitted from the first surface 141 is emitted to the reflecting film of the light-focusing structure 112, the light can be reflected by the reflecting film to have a tendency to converge towards a central region of a light field. In some embodiments, the light-emitting element 14 may also be an LED emitting light from five surfaces. The light-emitting element 14 may have four sides that are connected in sequence. The four sides are respectively connected to the first surface 141 and the second surface 142. The four sides and the first surface 141 may be the light-emitting surfaces of the light-emitting element 14. Light emitted from the four side surfaces can also be incident on the light-focusing structure 112, and is thus reflected by the reflecting film on the light-focusing structure 112 to have a tendency to converge towards the central region of the light field. The adoption of the light source that emits light on five sides as the light-emitting element 14 can increase output power of the light-emitting element 14, thereby increasing lighting illuminance of the distal end module 10 and satisfying a light supplementing requirement of the camera 13, and in cooperation with regulation and control effect of the light-focusing structure 112 and the reflecting film on distribution of the light field, lighting requirements of the camera 13 can be better met.

In the above distal end module 10, the light-focusing structure 112 and the reflecting film are arranged on the end cover 11 and cooperate to focus the light emitted by the light-emitting element 14, which can reduce a lighting beam angle of the distal end module 10 so that the lighting beam angle more closely conforms to the shooting field of view of the camera 13, thereby fully utilizing the light emitted by the light-emitting element 14 to supplement the camera 13 with light, so as to improve a light energy utilization efficiency. Meanwhile, the light is converged by the light-focusing structure 112 on the end cover 11. The light-focusing structure 112 is highly integrated with the distal end module 10, an arrangement process is simple, an arrangement cost is low, and a space occupied by the light-focusing structure 112 is small. Compared with structures such as a reflector, the light-focusing structure 112 can be better adapted to the distal end module 10 with a small size, thereby reducing an injury to a patient caused by the endoscope, while accommodating the focusing of a light-emitting element 14 having lower power in the distal end module 10. Furthermore, by gradually reducing the radial size of the light-focusing structure 112, the distribution of the light field can be effectively regulated and controlled, which is conducive to converging the light towards a center of the light field to improve central illuminance and a light output efficiency of the lighting light field, and meanwhile, while an edge field of view can maintain sufficient illuminance and good uniformity, thus effectively improving an illumination effect. The arrangement that the second surface 142 is higher than the second opening 1122 can also reduce the risk of the light emitted from the light-emitting element 14 leaking from the second opening 1122, which is also conducive to increasing the light output efficiency of the distal end module 10.

In some embodiments, the end cover 11 is further provided with a light-receiving hole 113. The camera 13 is arranged corresponding to the light-receiving hole 113 and can receive light from the lesion region via the light-receiving hole 113. The end cover 11 is provided with two light-focusing structures 112. The two light-focusing structures 112 are respectively arranged on two opposite sides of the light-receiving hole 113. The light-emitting element 14 is arranged in each of the two light-focusing structures 112, so as to supplement the camera 13 with light from two opposite sides of the camera 13, thereby improving shooting quality of the camera 13.

It can be understood that the beam angle of a single light-emitting element in a conventional distal end module is usually large, and when two ends of the camera are provided with the light-emitting elements respectively, a field angle of the light field formed by superimposing the beam angles of the two light-emitting elements is much greater than the shooting field of view of the camera, resulting in a significant amount of light being wasted because it falls to the field of view of the camera. In the distal end module 10 according to the present application, the light-focusing structure 112 and the reflecting film are arranged and cooperate to converge more light of the light-emitting elements 14 to the central region of the light field, which can effectively compress the beam angles of the light-emitting elements 14, so that the field angle of the light field formed by superimposing two light-emitting elements 14 is not excessively large, and can be well matched with the shooting field of view of the camera 13. which reduces a proportion of wasted light, thereby effectively improving the light energy utilization efficiency of the distal end module 10.

As shown in FIG. 1 and FIG. 3, in some embodiments, the distal end module 10 may further include a flexible circuit board 15, a bracket 16, a transmission line 17, and/or the like. The flexible circuit board 15 may be electrically connected to the photosensitive element 19 and the transmission line 17. The transmission line 17 extends into the handle and is electrically connected to a circuit board in the handle, so as to transmit an electric signal generated by the photosensitive element 19 to the circuit board. The bracket 16 can provide support and protection for the flexible circuit board 15, the transmission line 17, the photosensitive element 19, the camera 13, etc. Certainly, the distal end module 10 may further include any other suitable elements, and the above-mentioned elements may be also replaced by other elements, which may be specifically set according to requirements of the endoscope, and is not repeatedly described in the present application.

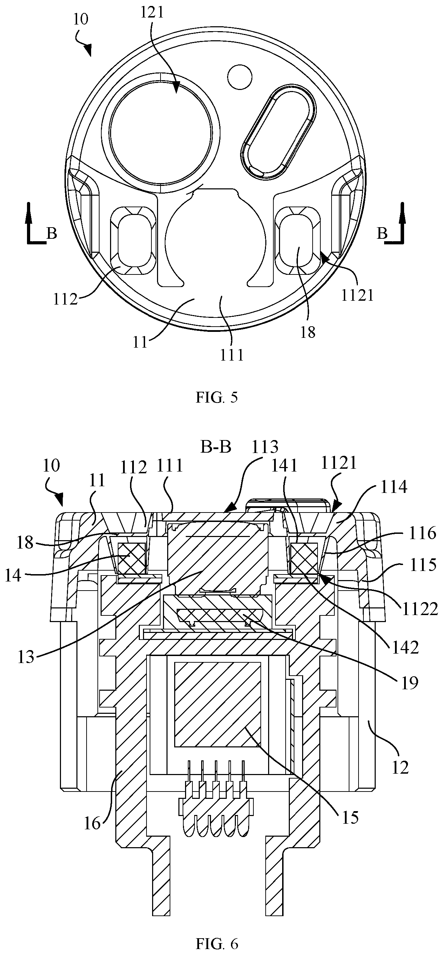

In the embodiment shown in FIG. 3, a distance between the first opening 1121 and the second opening 1122, i.e., a size of the light-focusing structure 112 in the axial direction, is equal to a thickness of the end cover 11. As shown in FIG. 5 and FIG. 6, the distal end modules 10 in the embodiment of FIG. 6 and the embodiment of FIG. 3 are mainly different in a shape and axial size of the light-focusing structure 112. In the embodiment shown in FIG. 6, the distance between the first opening 1121 and the second opening 1122 is greater than or equal to the thickness of the end cover 11. For example, in some embodiments, the end cover 11 has a top cover portion 114, a side cover portion 115, and a raised portion 116. The side cover portion 115 may be a part of a hollow cylindrical structure. The side cover portion 115 is connected to a side of the top cover portion 114 facing away from the end surface 111, and connected to a partial contour of the top cover portion 114. The raised portion 116 is connected to a side of the top cover portion 114 facing the side cover portion 115. The light-focusing structure 112 is positioned corresponding to the raised portion 116 and extends through the top cover portion 114 and the raised portion 116. In this embodiment, a size of the top cover portion 114 in the axial direction may be understood as the thickness of the end cover 11. The raised portion 116 is arranged to increase the size of the light-focusing structure 112 in the axial direction to cause the size of the light-focusing structure 112 in the axial direction to be greater than or equal to the thickness of the end cover 11, so that the risk that the light emitted by the light-emitting element 14 leaks from the second opening 1122 can be reduced, and meanwhile, an area of a surface of the light-focusing structure 112 provided with the reflecting film can also be increased, and therefore, the light-focusing structure 112 has a sufficiently large area to regulate and control the light emitted by the light-emitting element 14, so as to form a good lighting light field. In addition, the raised portion 116 is arranged on the side of the top cover portion 114 facing the side cover portion 115, so that while the axial size of the light-focusing structure 112 is increased, the first opening 1121 of the light-focusing structure 112 may be flush with the end surface 111 of the top cover portion 114 and does not protrude from the top cover portion 114, which is beneficial to reducing an occupied space of the distal end module 10 and improving appearance integrity of the distal end module 10.

Certainly, the reflecting film may cover not only the surface of the light-focusing structure 112, but in some embodiments, the reflecting film may also cover the surface of the end cover 11 corresponding to the first opening 1121, for example, cover the end surface 111 of the top cover portion 114 for facing the lesion region, and the reflecting film can also cover an outer circumferential surface of the side cover portion 115. A such, the reflecting film located outside the light-focusing structure 112 can reflect light emitted outside the light-focusing structure 112 of the end cover 11 towards the lesion region to improve the lighting effect on the lesion region, which is conducive to further improving the light energy utilization efficiency of the distal end module 10. A material and an arrangement process of the reflecting film are not limited. The material of the reflecting film includes, but is not limited to, any suitable metal material with good reflectivity such as aluminum, silver and chromium, and the reflecting film can be arranged on the end cover 11 by any suitable process such as sputtering.

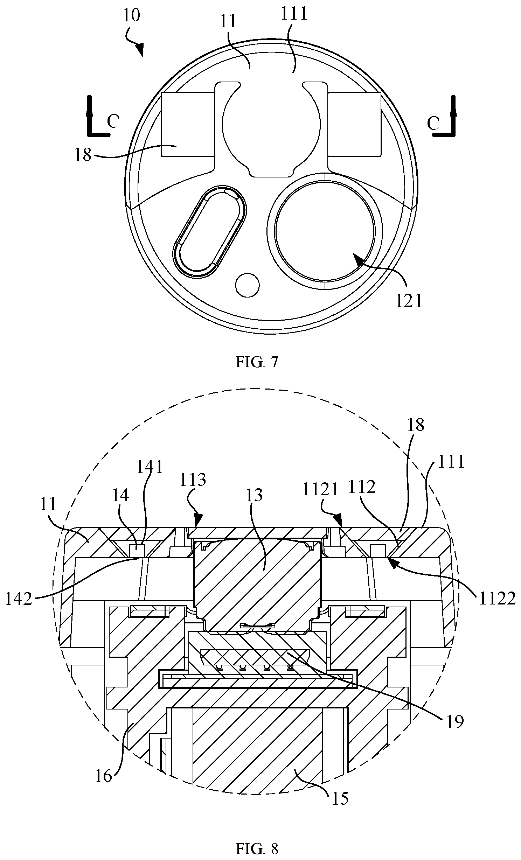

As shown in FIG. 2, FIG. 5, and FIG. 7, the distal end module 10 shown in FIG. 7 differs from the distal end modules shown in FIG. 2 and FIG. 5 mainly in the shape and axial size of the light-focusing structure 112. In some embodiments, a shape of a section of the light-focusing structure 112 on the plane perpendicular to the axial direction includes, but is not limited to, an irregular shape, an axisymmetric shape, a centrosymmetric shape or a rotationally symmetric shape. For example, in the embodiment shown in FIG. 2, the shape of the section of the light-focusing structure 112 on the plane perpendicular to the axial direction is a regular circle. In the embodiment shown in FIG. 5, the shape of the section of the light-focusing structure 112 on the plane perpendicular to the axial direction is an irregular shape such as an ellipse-like shape. In the embodiment shown in FIG. 7, the shape of the section of the light-focusing structure 112 on the plane perpendicular to the axial direction is a regular rectangle. The shape of the section of the light-focusing structure 112 on the plane perpendicular to the axial direction is not limited, and different sectional shapes have different regulation and control effects on the light field, as long as, in cooperation with parameters such as an inclination angle of the light-focusing structure 112 relative to the axial direction and the axial size of the light-focusing structure 112, good light field distribution can be obtained. For example, when the axial size and the inclination angle relative to the axial direction of the light-focusing structure 112 are constant, referring to FIG. 7 and FIG. 8, if the shape of the section of the light-focusing structure 112 on the plane perpendicular to the axial direction is a rectangle, the central illuminance of the light field resulting from superposing the two light-emitting elements 14 at a working distance of 50mm may reach 2907 Lux and is substantially increased by 169% compared to the case where the light-focusing structure 112 is not provided, and the edge illuminance at the working distance of 50 mm may reach 72 Lux. If the shape of the section of the light-focusing structure 112 on the plane perpendicular to the axial direction is a circle, the central illuminance of the light field resulting from superposed the two light-emitting elements 14 at the working distance of 50mm may reach 3758 Lux and is substantially increased by 218% compared to the case where the light-focusing structure 112 is not provided, the edge illuminance at the working distance of 50mm may reach 71.4 Lux, edge uniformity is reduced to 0.54%, and the edge uniformity of the light field without the light-focusing structure 112 at the working distance of 50 mm is usually greater than 10%. It can be seen that the circular or rectangular light-focusing structure 112 can effectively regulate and control the light field, and converge the light towards the central region of the light field, so as to better adapt to lighting of the camera 13.

In some embodiments, a shape of a section of the light-focusing structure 112 on a plane parallel to the axial direction is flat, for example, in the embodiments shown in FIG. 4, FIG. 6, and FIG. 8, the shape of the section of the light-focusing structure 112 on the plane parallel to the axial direction is flat. It can be understood that when the shapes of the sections of the light-focusing structure 112 on the plane perpendicular to the axial direction and the plane parallel to the axial direction are changed, the regulation and control effect of the light-focusing structure 112 on the light field is also changed, and in cooperation with the adjustment of the parameters such as the inclination angle of the light-focusing structure 112 relative to the axial direction, a good lighting light field can be obtained. For example, in some embodiments, the section of the light-focusing structure 112 on the plane parallel to the axial direction is flat, the section thereof on the plane perpendicular to the axial direction is rectangular, and an included angle between a contour of the section of the light-focusing structure 112 on the plane parallel to the axial direction and the axial direction (i.e., the inclination angle of the light-focusing structure 112 relative to the axial direction) is greater than or equal to 35° and less than or equal to 51°, so that the light-focusing structure 112 can effectively regulate and control the distribution of the light field. In some embodiments, the shape of the section of the light-focusing structure 112 on the plane parallel to the axial direction is flat, the shape of the section thereof on the plane perpendicular to the axial direction is circular, and the included angle between the contour of the section of the light-focusing structure 112 on the plane parallel to the axial direction and the axial direction is greater than or equal to 36.3°, so that the light-focusing structure 112 can also effectively regulate and control the distribution of the light field.

Different axial sizes of the light-focusing structure 112 (i.e., the distance between the first opening 1121 and the second opening 1122) may affect the area of the light-focusing structure 112 provided with the reflecting film, and also affect the regulation and control effect of the light-focusing structure 112 on the light. In some embodiments, the distance between the first opening 1121 and the second opening 1122 is greater than or equal to 0.5 mm and less than or equal to 2 mm. Therefore, it is conducive to causing the light-focusing structure 112 to have an enough area to reflect light to effectively regulate and control the distribution of the light field, and it can cause the axial size of the light-focusing structure 112 to be not excessively large, thereby facilitating reduction of the size of the distal end module 10.

Distribution effects of the light fields produced by the light-focusing structures 112 with different parameter settings are described in detail in the following embodiments.

In some embodiments, after the light field is regulated and controlled by the reflecting film arranged on the light-focusing structure 112, a ratio between the edge illuminance and the central illuminance of the light field resulting from superposing the two light-emitting elements 14, i.e., the lighting light field of the distal end module 10, is greater than or equal to 0.5% and less than or equal to 5%. Thus, the light-focusing structure 112 and the reflecting film can effectively converge the light towards the center of the light field to reduce the beam angle of the lighting light, so that the beam angle adapts to the shooting field of view of the camera 13, and meanwhile, sufficiently strong central illuminance is realized, and in addition, illuminance of the edge field of view is not excessively low, thereby effectively improving the good lighting effect.

In some embodiments, the size of the section of the light-emitting element 14 on the plane perpendicular to the axial direction is less than the size of the section of the second opening 1122 of the light-focusing structure 112 on the plane perpendicular to the axial direction. Therefore, a good regulation and control effect on the light field of the light-emitting element 14 can be achieved by the light-focusing structure 112 having a small area, which is beneficial to considering both the lighting effect and a miniaturization design of the distal end module 10.

Specific parameters and achieved lighting effects of the light-focusing structure 112 in some of the embodiments are exemplified below, and certainly, the specific parameter setting of the light-focusing structure 112 is not limited to the following examples.

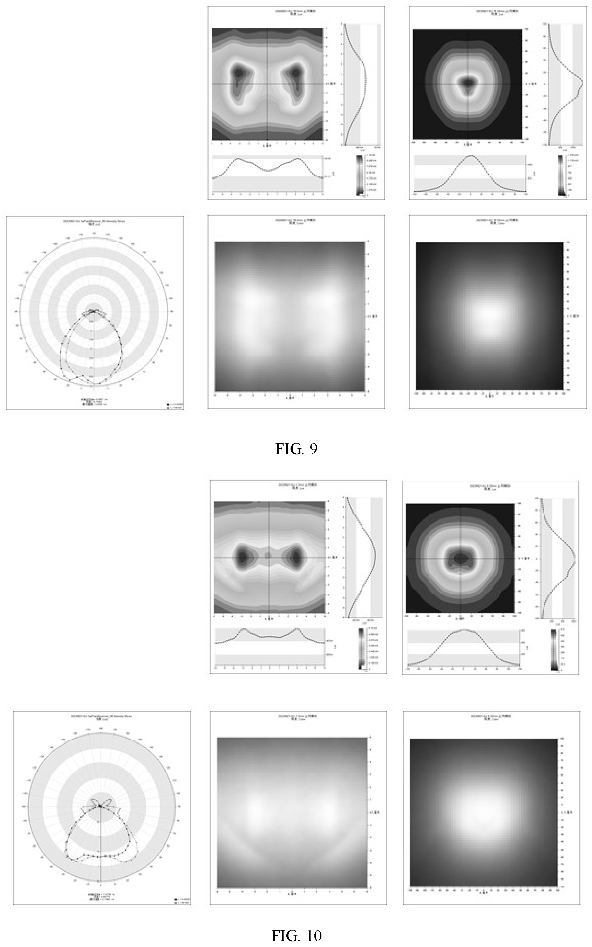

As shown in FIG. 5, FIG. 6 and FIG. 9, FIG. 9 shows diagrams of the lighting effect of the distal end module 10 in a first embodiment of the present application. In FIG. 9, a left diagram in the first row is an illuminance distribution diagram at a working distance of 3 mm, a right diagram in the first row is an illuminance distribution diagram at a working distance of 5 mm; in the second row, from left to right, a light distribution graph at 3 mm and 5 mm; a lighting light spot diagram at the working distance of 3 mm, and a lighting light spot diagram at the working distance of 50 mm are sequentially shown. In the first embodiment, the axial sizes of the light-focusing structures 112 are 1.8 mm. The shapes of the sections of the light-focusing structures 112 on the plane perpendicular to the axial direction are an asymmetric pattern which is substantially elliptical. The contours of the sections of the light-focusing structures 112 on the plane parallel to the axial direction are flat, the inclination angle of the light-focusing structures 112 relative to the axial direction is 48.15°. A size of the second opening 1122 is 1.65 mm*0.85 mm. A size of the first opening 1121 is 2.82 mm*1.70 mm. Coordinates of a central axis of one of the light-focusing structures 112 on the surface of the end cover 11 corresponding to the first opening 1121, i.e., the end surface 111 of the end cover 11 are (2.55, -2). The central axes of the two light-focusing structures 112 can be axisymmetric relative to the optical axis of the camera 13. A coordinate system on the end surface 111 of the end cover 11 takes the optical axis of the camera 13 as an origin, and takes a direction of a line connecting the central axes of the two light-emitting elements 14 as an X axis and a direction perpendicular to the line connecting the central axes of the two light-emitting elements 14 as a Y axis, which is applicable to other embodiments.

Referring to FIG. 10, FIG. 10 sequentially shows an illuminance distribution diagram at the working distance of 3 mm, an illuminance distribution diagram at the working distance of 50 mm, a light distribution graph at 3 mm and 50 mm, a lighting light spot diagram at the working distance of 3 mm, and a lighting light spot diagram at the working distance of 5 mm of a conventional distal end module without the light-focusing structure.

As can be seen from FIG. 9 and FIG. 10, in the first embodiment, the light output efficiency of the distal end module 10 is 70.9%, the beam angles in the X-axis direction and the Y-axis direction are 86° and 94° respectively, averages of the central illuminance and the edge illuminance at the working distance of 50 mm are 1354.42 Lux and 31.9 Lux respectively, and the edge uniformity at the working distance of 50 mm is 2.02%. Thus, through the regulation and control effect of the light-focusing structure 112 on the light field, the light output efficiency of the distal end module 10 is effectively improved, and the beam angle is reduced, thereby effectively improving the light energy utilization efficiency, so that it can be well adapted to the shooting field of view of the camber 13, and each of the central field of view and the edge field of view can have proper illuminance, which provides a good lighting effect. It should be noted that the conventional distal end module corresponding to FIG. 10 has the same light-emitting conditions as the distal end module 10 according to the first embodiment except that the light-focusing structure is not provided. For example, in the first embodiment, the axial size of the light-focusing structure 112 is greater than the thickness of the top cover portion 114, the second surface of the light-emitting element 14 is located on the side of the top cover portion 114 facing the side cover portion 115, and therefore, the second surface of the light-emitting element of the conventional distal end module corresponding to FIG. 10 is located on the side of the top cover portion facing the side cover portion.

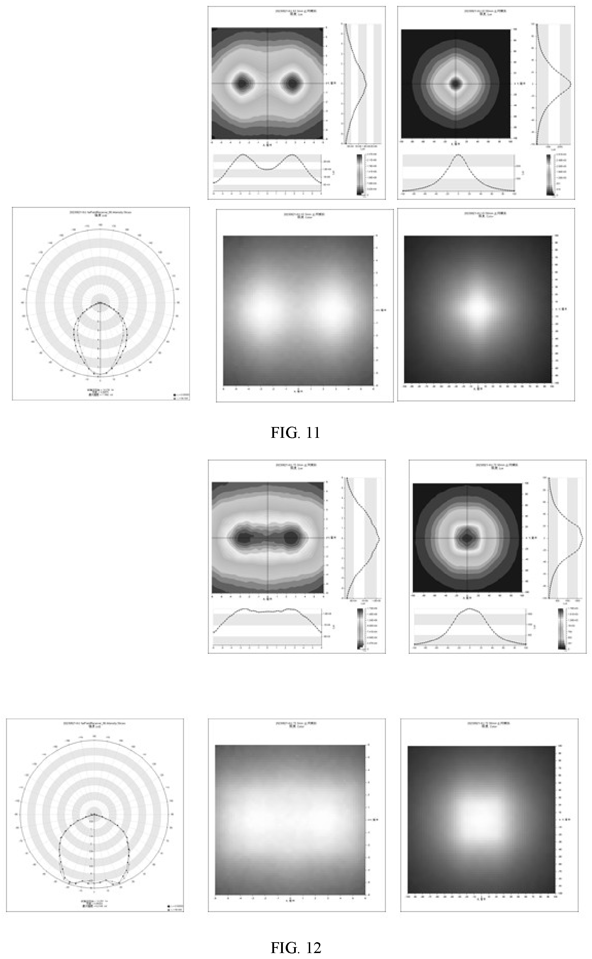

Referring to FIG. 11, FIG. 11 sequentially shows an illuminance distribution diagram at the working distance of 3 mm, an illuminance distribution diagram at the working distance of 50 mm, a light distribution graph at 3 mm and 50 mm, a lighting light spot diagram at the working distance of 3 mm, and a lighting light spot diagram at the working distance of 5 mm, of the distal end module 10 according to a second embodiment. In the second embodiment, the axial sizes of the light-focusing structures 112 are 0.7mm, the shapes of the sections of the light-focusing structures 112 on the plane perpendicular to the axial direction are a rectangle. The contours of the sections of the light-focusing structures 112 on the plane parallel to the axial direction are flat, the inclination angle of the light-focusing structures 112 relative to the axial direction is 36°. The size of the second opening 1122 is 0.9mm*0.55mm. The size of the first opening 1121 is 1.91mm*1.56mm. The coordinates of the central axis of one of the light-focusing structures 112 are (2.80, -2.07). In the second embodiment, the light output efficiency of the distal end module 10 is 89%, the beam angles in the X-axis direction and the Y-axis direction are 79° and 92° respectively, the averages of the central illuminance and the edge illuminance at the working distance of 50 mm are 2907.98 Lux and 72.62 Lux respectively, and the edge uniformity at the working distance of 50 mm is 0.54%.

Referring to FIG. 12, FIG. 12 sequentially shows an illuminance distribution diagram at the working distance of 3 mm, an illuminance distribution diagram at the working distance of 50 mm, a light distribution graph at 3 mm and 50 mm, a lighting light spot diagram at the working distance of 3 mm, and a lighting light spot diagram at the working distance of 5 mm, of the distal end module 10 according to a third embodiment. In the third embodiment, the axial sizes of the light-focusing structures 112 are 0.7mm, the shapes of the sections of the light-focusing structures 112 on the plane perpendicular to the axial direction are a rectangle. The contours of the sections of the light-focusing structures 112 on the plane parallel to the axial direction are flat, the inclination angle of the light-focusing structures 112 relative to the axial direction is 50.01°. The size of the second opening 1122 is 0.9mm*0.55mm, the size of the first opening 1121 is 2.57mm*2.22mm. The coordinates of the central axis of one of the light-focusing structures 112 are (2.80, -2.07). In the third embodiment, the light output efficiency of the distal end module 10 is 89.3%, the beam angles in the X-axis direction and the Y-axis direction are 113° and 112° respectively, the averages of the central illuminance and the edge illuminance at the working distance of 50 mm are 1758.45 Lux and 80.89 Lux respectively, and the edge uniformity at the working distance of 50 mm is 0.87%. As can be seen from FIG. 11 to FIG. 12, when the inclination angle of the light-focusing structure 112 relative to the axial direction ranges from 35° to 51°, the lighting beam angle of the distal end module 10 can be well matched with the shooting field of view of the camera 13, and a good lighting effect is obtained.

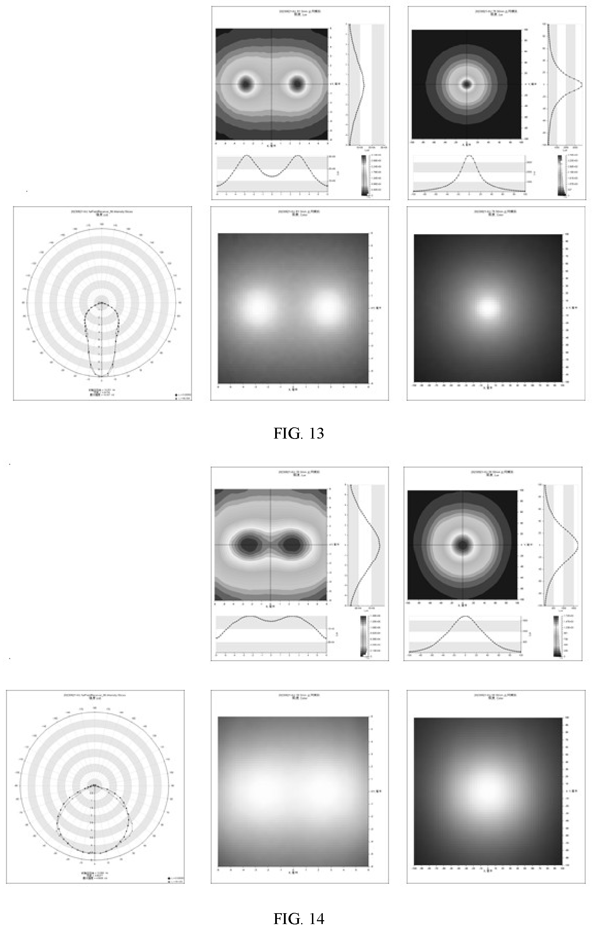

Referring to FIG. 13, FIG. 13 sequentially shows an illuminance distribution diagram at the working distance of 3 mm, an illuminance distribution diagram at the working distance of 50 mm, a light distribution graph at 3 mm and 50 mm, a lighting light spot diagram at the working distance of 3 mm, and a lighting light spot diagram at the working distance of 5 mm, of the distal end module 10 according to a fourth embodiment. In the fourth embodiment, the axial sizes of the light-focusing structures 112 are 0.7 mm, the shapes of the sections of the light-focusing structures 112 on the plane perpendicular to the axial direction are a circle, the contours of the sections of the light-focusing structures 112 on the plane parallel to the axial direction are flat, the inclination angle of the light-focusing structures 112 relative to the axial direction is 36.29°. A radial size of the second opening 1122 is 0.9 mm, a radial size of the first opening 1121 is 1.928 mm. The coordinates of the central axis of one of the light-focusing structures 112 are (2.80, -2.07). In the fourth embodiment, the light output efficiency of the distal end module 10 is 88.8%, the beam angles in the X-axis direction and the Y-axis direction are 44° and 48° respectively, the averages of the central illuminance and the edge illuminance at the working distance of 50 mm are 3758.74 Lux and 71.49 Lux respectively, and the edge uniformity at the working distance of 50 mm is 0.72%.

Referring to FIG. 14, FIG. 14 shows diagrams of the lighting effect of a conventional distal end module 10 without the light-focusing structure 112. It should be noted that FIG. 10 and FIG. 14 respectively show diagrams of the lighting effects of two conventional distal end modules without the light-focusing structure, where the distal end module corresponding to FIG. 14 has the same light-emitting conditions as the distal end module 10 in the fourth embodiment except that the light-focusing structure is not provided. For example, in the fourth embodiment, the axial size of the light-focusing structure 112 is equal to the thickness of the top cover portion 114, the light-emitting element 14 is arranged in the top cover portion 114, and therefore, the light-emitting element of the conventional distal end module corresponding to FIG. 14 is also located in the top cover portion. FIG. 14 sequentially shows an illuminance distribution diagram at the working distance of 3 mm, an illuminance distribution diagram at the working distance of 50 mm, a light distribution graph at 3 mm and 50 mm, a lighting light spot diagram at the working distance of 3 mm, and a lighting light spot diagram at the working distance of 5 mm, of the conventional distal end module. The light output efficiency of the conventional distal end module is 85.4%, the beam angles in the X-axis direction and the Y-axis direction are 111° and 120° respectively, the averages of the central illuminance and the edge illuminance at the working distance of 50 mm are 1716.20 Lux and 71.31 Lux respectively, and the edge uniformity at the working distance of 50 mm is 10.31%. As can be seen from FIG. 13 and FIG. 14, compared with the conventional distal end module, in the distal end module 10 according to the fourth embodiment of the present application, through the regulation and control effect of the light-focusing structure 112 on the light field, the light output efficiency of the distal end module 10 is effectively improved, and the beam angle is reduced, thereby effectively improving the light energy utilization efficiency. Moreover, it can be well adapted to the shooting field of view of the camber 13, and each of the central field of view and the edge field of view has proper illuminance, which provides a good lighting effect.

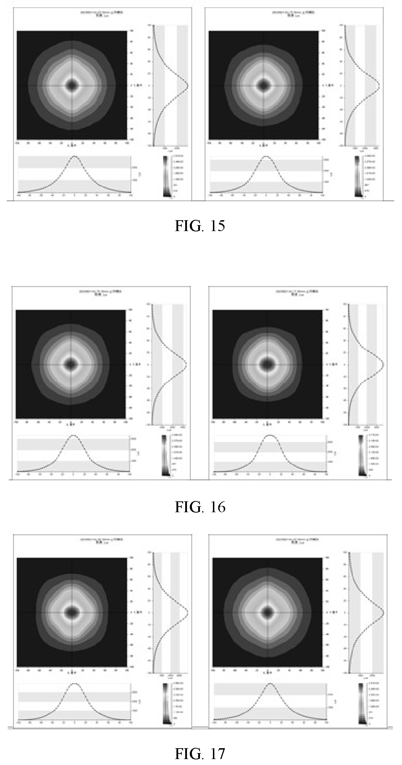

It can be appreciated that a change in the size of the light-emitting element 14 may affect the distribution and illuminance value of the light field emitted from the light-emitting surface of the light-emitting element 14. Referring to FIG. 15, FIG. 15 schematically illustrates a comparison of the lighting effects of two embodiments in which the sizes of the light-emitting element 14 are different. In FIG. 15, for the embodiment corresponding to the right diagram, the size of the section of the light-emitting element 14 on the plane perpendicular to the axial direction is reduced by half compared with the embodiment corresponding to the left diagram. The left diagram of FIG. 15 shows an illuminance distribution diagram of the distal end module 10 in a fifth embodiment at the working distance of 50 mm. In the fifth embodiment, the light-focusing structure 112 has an axial size of 0.7 mm, and a rectangular sectional shape, the inclination angle of the light-focusing structure 112 relative to the axial direction is 36°. The size of the second opening 1122 is 0.9mm*0.55mm, the size of the first opening 1121 is 1.91mm*1.56mm, and the coordinates of the central axis of the light-focusing structure 112 are (2.8, -2.07). In the fifth embodiment, the light output efficiency of the distal end module 10 is 89%, the beam angles in the X-axis direction and the Y-axis direction are 79° and 92° respectively, the averages of the central illuminance and the edge illuminance at the working distance of 50 mm are 2907.98 Lux and 72.62 Lux respectively, and the edge uniformity at the working distance of 50 mm is 0.54%. The right diagram of FIG. 15 shows an illuminance distribution diagram of the distal end module 10 in a sixth embodiment at the working distance of 50 mm. In the sixth embodiment, the light-focusing structure 112 has an axial size of 0.7 mm, and a rectangular sectional shape, the inclination angle of the light-focusing structure 112 relative to the axial direction is 36°. The size of the second opening 1122 is 0.45mm*0.28mm, the size of the first opening 1121 is 1.47mm*1.29mm, and the coordinates of the central axis of the light-focusing structure 112 are (2.8, -2.07). In the sixth embodiment, the light output efficiency of the distal end module 10 is 89%, the beam angles in the X-axis direction and the Y-axis direction are 79° and 92° respectively, the averages of the central illuminance and the edge illuminance at the working distance of 50 mm are 3350.94 Lux and 54.61Lux respectively, and the edge uniformity at the working distance of 50 mm is 0.98%. As can be seen from FIG. 15, when the size of the light-emitting element 14 changes, the illuminance distribution of the distal end module 10 also changes, but the lighting effect can be improved.

It can be appreciated that a change in a height of the light-emitting element 14, i.e., the axial size of the light-emitting element 14 may also affect the distribution and illuminance value of the light field emitted from the light-emitting surface of the light-emitting element 14. Referring to FIG. 16, FIG. 16 illustrates a comparison of the lighting effects of two embodiments in which the heights of the light-emitting element 14 are different. In FIG. 16, for the embodiment corresponding to the right diagram, the axial size of the light-emitting element 14 is reduced by half compared with the embodiment corresponding to the left diagram. The left diagram in FIG. 16 corresponds to the distal end module 10 according to the sixth embodiment, in which the parameter setting and the lighting effect of the light-focusing structure 112 are the same as those of the sixth embodiment. The right diagram of FIG. 16 shows an illuminance distribution diagram of the distal end module 10 according to a seventh embodiment at the working distance of 50 mm. In the seventh embodiment, the light-focusing structure 112 has an axial size of 0.7mm, and a rectangular sectional shape, the inclination angle of the light-focusing structure 112 relative to the axial direction is 36°, the size of the second opening 1122 is 0.45mm*0.28mm, the size of the first opening 1121 is 1.47mm*1.29mm, and the coordinates of the central axis of the light-focusing structure 112 are (2.8, -2.07). In the seventh embodiment, the light output efficiency of the distal end module 10 is 88.2%, the beam angles in the X-axis direction and the Y-axis direction are 61° and 79° respectively, the averages of the central illuminance and the edge illuminance at the working distance of 50 mm are 3706.28 Lux and 29.17 Lux respectively, and the edge uniformity at the working distance of 50 mm is 0.6%. As can be seen from FIG. 16, when the axial size of the light-emitting element 14 changes, the illuminance distribution of the distal end module 10 also changes, but the lighting effect can be improved.

In some embodiments, the distal end module 10 further includes a light-transmitting sealing structure 18. The light-transmitting sealing structure 18 is arranged in the light-focusing structure 112 and seals a side of the light-emitting element 14 facing the first opening 1121. For example, the light-transmitting sealing structure 18 may be a light-transmitting flat plate structure or a structure with a curved surface arranged between the first surface 141 of the light-emitting element 14 and the first opening 1121 of the light-focusing structure 112. The light-transmitting sealing structure 18 may also be partially arranged around the side of the light-emitting element 14 along its circumferential direction, and may wrap the side and the first surface 141 of the light-emitting element 14, or partially extend to the second surface 142 of the light-emitting element 14, as long as the side of the light-emitting element 14 facing the first opening 1121 can be sealed. A material of the light-transmitting sealing structure 18 includes, but is not limited to, a material with transmittance greater than or equal to 90% and good biocompatibility, so that light output of the light-emitting element 14 is not affected, and the patient is not prone to be damaged. The light-transmitting sealing structure 18 is arranged to seal the light-emitting element 14, so that the light emitted by the light-emitting element 14 can be deflected while the light-emitting element 14 and internal elements of the distal end module 10 are protected from water, and in cooperation with the arrangement of the light-focusing structure 112 and the reflecting film, the distribution of the light field can be better regulated and controlled to improve the lighting effect.

Referring to FIG. 17, the embodiment corresponding to the right diagram of FIG. 17 has the light-transmitting sealing structure 18 compared with the embodiment corresponding to the left diagram, and other setting conditions are the same. In FIG. 17, the left diagram shows an illuminance distribution diagram of the distal end module 10 according to an eighth embodiment at the working distance of 50 mm. In the eighth embodiment, the light-focusing structure 112 has an axial size of 0.7 mm, and a rectangular sectional shape, the inclination angle of the light-focusing structure 112 relative to the axial direction is 36°, the size of the second opening 1122 is 0.9mm*0.55mm, the size of the first opening 1121 is 1.91mm*1.56mm, and the coordinates of the central axis of the light-focusing structure 112 are (2.8, -2.07). In the eighth embodiment, the light output efficiency of the distal end module 10 is 96.9%, the beam angles in the X-axis direction and the Y-axis direction are 65° and 82° respectively, the averages of the central illuminance and the edge illuminance at the working distance of 50 mm are 3956.88Lux and 46.78 Lux respectively, and the edge uniformity at the working distance of 50 mm is 0.93%. The right diagram of FIG. 17 shows an illuminance distribution diagram of the distal end module 10 according to a ninth embodiment at the working distance of 50 mm. In the ninth embodiment, the light-focusing structure 112 has an axial size of 0.7 mm, and a rectangular sectional shape, the inclination angle of the light-focusing structure 112 relative to the axial direction is 36°, the size of the second opening 1122 is 0.9mm*0.55mm, the size of the first opening 1121 is 1.91mm*1.56mm, and the coordinates of the central axis of the light-focusing structure 112 are (2.8, -2.07). In the ninth embodiment, the light output efficiency of the distal end module 10 is 89%, the beam angles in the X-axis direction and the Y-axis direction are 79° and 92° respectively, the averages of the central illuminance and the edge illuminance at the working distance of 50 mm are 2907.98 Lux and 72.62 Lux respectively, and the edge uniformity at the working distance of 50 mm is 0.54%. It can be seen from FIG. 17 that, when the light-transmitting sealing structure 18 is provided, a difference between the edge illuminance and the central illuminance is smaller due to the deflection effect of the light-transmitting sealing structure 18 on the light, which is beneficial to improving the lighting effect.

In some embodiments, the section of the light-focusing structure 112 on the plane parallel to the axial direction may also be a curved surface such as a concave arc surface. The light-focusing structure 112 with the concave arc surface has a stronger light-focusing effect, and can be applied to embodiments in which the sectional size of the light-emitting element 14 is relatively small, or an occupied space of the first opening 1121 and the second opening 1122 of the light-focusing structure 112 on the top cover portion 114 is relatively small. A stronger light field regulation and control effect is achieved by the light-focusing structure 112 with a relatively small occupied space. An example is given below in combination with structures of the end cover 11 and the sleeve 12 and the occupied space of the light-focusing structure 112.

Referring to FIG. 1, in some embodiments, the top cover portion 114 is substantially a part of a circular plate-like structure, an end portion of the sleeve 12 is substantially another part of the circular plate-like structure, and the end portion of the sleeve 12 and the top cover portion 114 are in butt connection to form the substantially complete circular plate-like structure. The end cover 11 may be made of a transparent material such as a transparent plastic material, which is beneficial to reducing influences of the end cover 11 on light emission of the light-emitting element 14 and light receiving of the camera 13. The sleeve 12 may be made of an opaque material such as an opaque plastic material, so as to shield the flexible circuit board 15, the photosensitive element 19, or the like, in the distal end module. Since the end portion of the sleeve 12 needs to reserve an instrument hole 121 for a medical instrument to extend through. The end portion of the sleeve 12 can reserve other parts to meet use requirements of other elements, which leads to that the arrangement area of the top cover portion 114 is limited, and the space of the top cover portion 114 for receiving the light-focusing structure 112 is also limited. It should be noted that, in order to adapt to the arrangement of the light-emitting element 14 near the second opening 1122 and the small size limitation of the distal end module 10, an adjustable range of the size of the second opening 1122 is generally relatively small, an adjustable range of the first opening 1121 is larger. Therefore, in the present application, a proportion of the size of the light-focusing structure on the end surface 111 is represented by the ratio between the radial size of the first opening 1121 and the radial size of the second opening 1122.

In some embodiments, if the first opening 1121 of the light-focusing structure 112 occupies a relatively small space on the top cover portion 114, for example, when the ratio of the radial size of the first opening 1121 to the radial size of the second opening 1122 ranges from 1.55 to 2, for example, 1.77, a concave curvature of the contour of the section of the light-focusing structure 112 on the plane parallel to the axial direction may be greater than or equal to 1.5 and less than or equal to 10. The light-focusing structure 112 is controlled to regulate and control the light by providing the concave arc surface, and the range setting of the concave curvature can also adapt to the relatively small occupied space of the first opening 1121, balance proportions of light in the center of the light field and edge light, and thereby obtaining a good lighting light field. In some embodiments, if the first opening 1121 of the light-focusing structure 112 occupies a moderate space on the top cover portion 114, for example, when the ratio of the radial size of the first opening 1121 to the radial size of the second opening 1122 ranges from 2.1 to 2.34, for example, 2.22, the concave curvature of the contour of the section of the light-focusing structure 112 on the plane parallel to the axial direction may be greater than or equal to 1 and less than or equal to 2.8. If the first opening 1121 of the light-focusing structure 112 occupies a larger space on the top cover portion 114, for example, when the ratio of the radial size of the first opening 1121 to the radial size of the second opening 1122 ranges from 2.35 to 2.58, for example, 2.46, the concave curvature of the contour of the section of the light-focusing structure 112 on the plane parallel to the axial direction may be greater than or equal to 2 and less than or equal to 5. The light-focusing structure 112 is controlled to regulate and control the light by providing the concave arc surface, and the range setting of the concave curvature can also adapt to the relatively small occupied space of the first opening 1121, balance the proportions of the light in the center of the light field and the edge light, and thereby obtaining a good lighting light field.

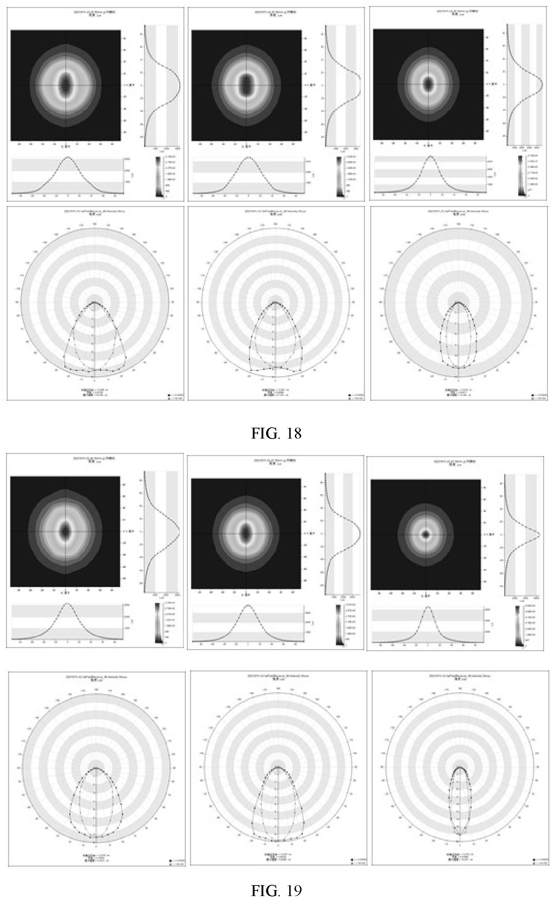

Several embodiments in which the concave curvature of the contour of the section of the light-focusing structure 112 on the plane parallel to the axial direction is different are given below as examples. Referring to FIG. 18, the first row of FIG. 18 respectively, shows from left to right, illuminance distribution diagrams at a working distance of 50 mm of the distal end module 10 according to a tenth embodiment, an eleventh embodiment, and a twelfth embodiment , and the second row respectively shows, from left to right, light distribution diagrams at the working distance of 50 mm of the distal end module 10 according to the tenth embodiment, the eleventh embodiment, and the twelfth embodiment. In the tenth embodiment, the eleventh embodiment, and the twelfth embodiment, the ratio of the radial size of the first opening 1121 to the radial size of the second opening 1122 is 1.77, the axial size of the light-focusing structure 112 is 0.7 mm, the shape of the section of the light-focusing structure 112 on the plane perpendicular to the axial direction is a circle, the light-focusing structure 112 is inclined by 43° relative to the axial direction, a diameter of the second opening 1122 is 0.9 mm, a diameter of the first opening 1121 is 1.78 mm, and the coordinates of the central axis of the light-focusing structure 112 are (2.8, -2.07). The three embodiments differ in that the concave curvatures of the contours of the sections of the light-focusing structures 112 on the plane parallel to the axial direction in the tenth embodiment, the eleventh embodiment and the twelfth embodiment are 1.5, 1.8 and 10 respectively.

Referring to FIG. 19, the first row of FIG. 19 respectively shows, from left to right, illuminance distribution diagrams at a working distance of 50 mm of the distal end module 10 according to a thirteenth embodiment, a fourteenth embodiment, and a fifteenth embodiment, and the second row respectively shows, from left to right, light distribution diagrams at the working distance of 50 mm of the distal end module 10 according to the thirteenth embodiment, the fourteenth embodiment, and the fifteenth embodiment. In the thirteenth embodiment, the fourteenth embodiment, and the fifteenth embodiment, the ratio of the radial size of the first opening 1121 to the radial size of the second opening 1122 is 2.22, the axial size of the light-focusing structure 112 is 0.7 mm, the shape of the section of the light-focusing structure 112 on the plane perpendicular to the axial direction is a circle, the light-focusing structure 112 is inclined by 43° relative to the axial direction, a diameter of the second opening 1122 is 0.9 mm, a diameter of the first opening 1121 is 2 mm, and the coordinates of the central axis of the light-focusing structure 112 are (2.8, -2.07). The three embodiments differ in that the concave curvatures of the contours of the sections of the light-focusing structures 112 on the plane parallel to the axial direction in the thirteenth embodiment, the fourteenth embodiment, and the fifteenth embodiment are 1, 1.3 and 2.8 respectively.

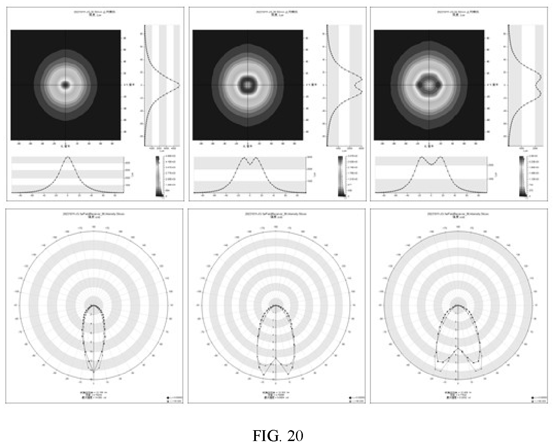

Referring to FIG. 20, the first row of FIG. 20 respectively shows, from left to right, illuminance distribution diagrams at a working distance of 50 mm of the distal end module 10 according to a sixteenth embodiment, a seventeenth embodiment and an eighteenth embodiment, and the second row respectively shows, from left to right, light distribution diagrams at the working distance of 50 mm of the distal end module 10 according to the sixteenth embodiment, the seventeenth embodiment and the eighteenth embodiment. In the sixteenth embodiment, the seventeenth embodiment and the eighteenth embodiment, the ratio of the radial size of the first opening 1121 to the radial size of the second opening 1122 is 2.46, the axial size of the light-focusing structure 112 is 0.7 mm, the shape of the section of the light-focusing structure 112 on the plane perpendicular to the axial direction is a circle, the light-focusing structure 112 is inclined by 43° relative to the axial direction, a diameter of the second opening 1122 is 0.9 mm, a diameter of the first opening 1121 is 2.2 mm, and the coordinates of the central axis of the light-focusing structure 112 are (2.8, -2.07). The three embodiments differ in that the concave curvatures of the contours of the sections of the light-focusing structures 112 on the plane parallel to the axial direction in the sixteenth embodiment, the seventeenth embodiment and the eighteenth embodiment are 2, 2.8 and 5 respectively.

In the above embodiments according to the present application, by the regulation and control effect of the light-focusing structure 112 and the reflecting film on the light field, the beam angle of the distal end module 10 can well adapt to the shooting field of view of the camera 13, and meanwhile, the distribution of the light field is optimized, and the lighting effect is improved. When the parameters such as the axial size and the sectional shape of the light-focusing structure 112, the axial size and the sectional shape of the light-emitting element 14, etc., are changed, the distribution of the light field can be adjusted to meet different lighting requirements.

The technical features in the above embodiments may be combined arbitrarily. For concise description, not all possible combinations of the technical features in the above embodiments are described. However, provided that they do not conflict with each other, all combinations of the technical features are to be considered to be within the scope of protection of the present application.

The above-mentioned embodiments only illustrate several implementations of the present application, and their description is specific and detailed, but should not be understood as a limitation on the protection scope of the present application. It should be noted that, for a person of ordinary skill in the art, various variants and improvements can be further made without departing from the conception of the present application, and these all fall within the protection scope of the present application. Therefore, the protection scope of the present application should be subject to the appended claims.

## Claims

1. A distal end module used in an endoscope, comprising:
an end cover provided with a light-focusing structure extending through the end cover, a first opening and a second opening being formed at two ends of the light-focusing structure respectively, a radial size of the light-focusing structure being gradually reduced in a direction from the first opening to the second opening, and a reflecting film being arranged on a surface of the light-focusing structure; and
a light-emitting element arranged in the light-focusing structure and having a first surface facing the first opening and a second surface facing the second opening, the second surface being higher than the second opening in a direction from the second opening to the first opening.

2. The distal end module according to claim 1, wherein a distance between the first opening and the second opening is greater than or equal to a thickness of the end cover.

3. The distal end module according to claim 1, wherein the end cover has a top cover portion, a side cover portion and a raised portion, wherein the side cover portion is connected to a side of the top cover portion and connected to a part of a contour of the top cover portion, the raised portion is connected to a side of the top cover portion facing the side cover portion, and the light-focusing structure is positioned corresponding to the raised portion and extends through the top cover portion and the raised portion.

4. The distal end module according to claim 1, wherein a shape of a section of the light-focusing structure on a plane perpendicular to an axial direction of the distal end module is an irregular shape, an axisymmetric shape, a centrosymmetric shape or a rotationally symmetric shape.

5. The distal end module according to claim 1, wherein a shape of a section of the light-focusing structure on a plane parallel to an axial direction of the distal end module is a flat surface a curved surface.

6. The distal end module according to claim 1, wherein a shape of a section of the light-focusing structure on a plane parallel to an axial direction of the distal end module is flat, a shape of a section of the light-focusing structure on a plane perpendicular to the axial direction is rectangular, and an included angle between a contour of the section of the light-focusing structure on the plane parallel to the axial direction and the axial direction is greater than or equal to 35° and less than or equal to 51°; or,
the shape of the section of the light-focusing structure on the plane parallel to the axial direction is flat, the shape of the section of the light-focusing structure on the plane perpendicular to the axial direction is circular, and the included angle between the contour of the section of the light-focusing structure on the plane parallel to the axial direction and the axial direction is greater than or equal to 36.3°.

7. The distal end module according to claim 1, wherein a shape of a section of the light-focusing structure on a plane parallel to an axial direction of the distal end module is a concave arc surface, and when a ratio between a radial size of the first opening and a radial size of the second opening ranges from 1.55 to 2, a curvature of a contour of the section of the light-focusing structure on the plane parallel to the axial direction is greater than or equal to 1.5 and less than or equal to 10; when the ratio between the radial size of the first opening and the radial size of the second opening ranges from 2.1 to 2.34, the curvature of the contour of the section of the light-focusing structure on the plane parallel to the axial direction is greater than or equal to 1 and less than or equal to 2.8; when the ratio between the radial size of the first opening and the radial size of the second opening ranges from 2.35 to 2.58, the curvature of the contour of the section of the light-focusing structure on the plane parallel to the axial direction is greater than or equal to 2 and less than or equal to 5.

8. The distal end module according to claim 1, wherein a distance between the first opening and the second opening is greater than or equal to 0.5 mm and less than or equal to 2 mm.

9. The distal end module according to claim 1, wherein a ratio of edge illuminance to central illuminance, at a working distance, of a lighting light field of the light-emitting element passing through the light-focusing structure is greater than or equal to 0.5% and less than or equal to 5%.

10. The distal end module according to claim 1, wherein a size of a section of the light-emitting element on a plane perpendicular to an axial direction of the distal end module is less than a size of a section of the second opening of the light-focusing structure on the plane perpendicular to the axial direction.

11. The distal end module according to claim 1, wherein the reflecting film covers a surface of the light-focusing structure and a surface of the end cover corresponding to the first opening; and/or
a material of the reflecting film comprises aluminum, silver or chromium.

12. The distal end module according to claim 1, wherein the distal end module further comprises a camera, the end cover is further provided with a light-receiving hole, the camera is arranged corresponding to the light-receiving hole, the end cover is provided with two light-focusing structures, the two light-focusing structures being arranged on two opposite sides of the light-receiving hole respectively, and the light-emitting element is arranged in each of the two light-focusing structures.

13. The distal end module according to claim 1, wherein the distal end module further comprises a light-transmitting sealing structure, the light-transmitting sealing structure being arranged in the light-focusing structure and sealing a side of the light-emitting element facing the first opening.

14. An endoscope, comprising an insertion portion and the distal end module according to any one of claims 1 to 13, the distal end module being arranged at an end portion of the insertion portion.
